# EUROPEAN PATENT APPLICATION

(11) **EP 1 356 814 A1**
(43) Date of publication of application: **29.10.2003**
(21) Application number: 01272815.0
(22) Date of filing: 17.12.2001
(51) Int. Cl.: A61K 31/41, A61K 9/107, A61K 47/14, A61K 47/24, A61K 47/44

(54) **PHARMACEUTICAL EMULSION PREPARATION**

(30) Priority: 28.12.2000 JP 2000400130; 22.01.2001 JP 2001013160
(71) Applicant: DAIICHI PHARMACEUTICAL CO., LTD., Chuo-ku, Tokyo 103-8234 (JP)
(72) Inventor: EBIHARA, K., c/o Daiichi Pharmaceutical Co., Ltd., Edogawa-ku, Tokyo 134-8630 (JP); SUZUKI, N., c/o Daiichi Pharmaceutical Co., Ltd., Edogawa-ku, Tokyo 134-8630 (JP); YAMAUCHI, H., c/o Daiichi Pharmaceutical Co., Ltd., Takatsuki-shi, Osaka 569-0806 (JP); KIKUCHI, H., c/o Daiichi Pharmaceutical Co., Ltd., Edogawa-ku, Tokyo 134-8630 (JP)
(74) Representative: Kinzebach, Werner, Dr.
(86) International application number: JP0111047
(87) International publication number: WO02053154

(57) **Abstract**

A stable and high-quality emulsion preparation containing ebselen can be obtained by using phosphatidylcholine, adding a predetermined amount of phosphatidylethanolamine thereto, and adjusting the pH of the final preparation to 6-8.

## Description

### TECHNICAL FIELD

The present invention relates to an ebselen-containing emulsion preparation having good stability and qualities.

### BACKGROUND ART

Ebselen is a compound having the following structural formula: 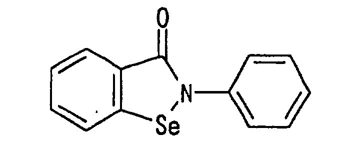 and at present this compound is under development as a preventive and therapeutic agent against cerebral disorders (see JP-A 1-131113 (the term "JP-A" as used herein means an "unexamined published Japanese patent application").

Ebselen is a compound having a very low solubility in water, and for development thereof as an injection, ebselen has been dissolved in an organic solvent to form a pharmaceutical preparation, but there was the problem that when the pharmaceutical preparation is diluted with water or physiological saline to inject it, crystals of ebselen are precipitated. Accordingly, a method of obtaining a stable aqueous solution by mixing ebselen with one or more natural or synthetic phospholipids was invented (see JP-A 2-250876), but there were cases where ebselen is decomposed or separated into two layers or a color of the resulting preparation is changed.

The conventional preparations were poor in stability and not satisfactory in respect of qualities.

### DISCLOSURE OF THE INVENTION

As a result of intensive studies under these circumstances, the present inventors found that a stable and high-quality emulsion preparation containing ebselen can be obtained by using phosphatidylcholine (also referred to hereinafter as PC) , adding a predetermined amount of phosphatidylethanolamine (also referred to hereinafter as PE) thereto and adjusting the pH of the final preparation to 6-8, and the present invention was thereby completed.

The phosphatidylcholine is preferably the one derived from lecithin, and the lecithin includes egg yolk lecithin, soybean lecithin etc., among which egg yolk lecithin etc. are preferable.

In the present invention, lecithin can also be used in place of PC, and in this case, the content of phosphatidylcholine in lecithin is preferably 90 % or more, more preferably 98 % or more.

The ratio of phosphatidylcholine and phosphatidylethanolamine is usually phosphatidylethanolamine : phosphatidylcholine = 0.1 99.9 to 10 : 90 (weight ratio) , preferably phosphatidylethanolamine : phosphatidylcholine = 0.1 : 99.9 to 5: 95. The concentration of lecithin in the final preparation is 0.5 to 4 %.

The fat and oil used are preferably soybean oil, corn oil, peanut oil, safflower oil, olive oil, sesame oil, castor oil, cottonseed oil, cacao fat, hardened oil, tallow, egg yolk oil, and triglycerides such as middle-chain fatty acid triglycerides. In particular, vegetable oils such as soybean oil, corn oil and safflower oil are preferable, and particularly soybean oil is preferable.

The content of the fat and oil/100 ml emulsion preparation is usually 1 to 40 g, preferably 5 to 30 g.

The pH value of the preparation is preferably from 6 to 8, more preferably from 6.5 to 7 . 5 . The pH value may be adjusted with sodium hydroxide, diethanolamine, triethanolamine, tromethamine, glycine, ammonia etc.

Although the content of ebselen is varied depending on the fat and oil used, the content is usually 0.1 to 2 mg per ml of fat and oil, and the concentration thereof in the final preparation is 0.01 to 0.2 %.

The preparation of the present invention may not be rendered isotonic, but may be rendered isotonic by sugars such asmannitol, galactose, glucose, inositol, lactose and sucrose, polyols such as glycerin, propylene glycol, butylene glycol and ethylene glycol, and isotonicity-conferring agents such as sodium chloride.

Further, suitable additives can be used in pharmaceutical preparation, and the additives include antioxidants such as vitamin C, vitamin E, BHT and sodium sulfite, stabilizers such as lysolecithin, oleic acid, stearic acid, dextrin and cyclodextrin, and anesthetics such as procaine hydrochloride.

The particle diameter of the emulsion of the present invention is preferably 100 nm to 300 nm from the viewpoint of hepatic toxicity etc.

The emulsion preparation of the present invention can be prepared in the following steps.

Ebselen is dissolved in fat and oil having phosphatidylcholine and phosphatidylethanolamine dispersed or dissolved therein, or phosphatidylcholine and phosphatidylethanolamine are dispersed or dissolved in fat and oil having ebselen dissolved therein. To the resulting solution is added water or an aqueous solution having additives such as glycerin dissolved therein, and the mixture is preliminarily emulsified by a homogenizer, and after the pH is adjusted, it is emulsified in a high-pressure emulsifier or a high-pressure jetting-type emulsifier. After the pH value of the resulting emulsion is adjusted, the emulsion is pipetted into the vial and sterilized, whereby the desired O/W emulsion preparation can be obtained.

As the method of sterilization, filtration or heat sterilization can be used. In the case of filtration , the emulsion preparation may be adjusted to pH 6-8. In the case of heat sterilization, the emulsion preparation before sterilization may be adjusted to pH 8-9, and upon sterilization thereof, the desired emulsion preparation having pH 6 to 8 can be formed.

### BEST MODE FOR CARRYING OUT THE INVENTION

### EXAMPLE 1

① 50 g conc. glycerin was accurately weighed, and distilled water for injection was added thereto to adjust the total volume to 2000 ml.
② 1.485 g of egg yolk lecithin (phosphatidylcholine content: 98%) and 0.015 g phosphatidylethanolamine were weighed and placed in an egg-plant type conical beaker, and dissolved by adding chloroform. Then, the chloroform was distilled away in an evaporator, and the sample was dried overnight under reduced pressure in a desiccator to give a lipid mixture.
③ Separately, 0.2 g ebselen and 50 g soybean oil were weighed and placed in another vessel and treated in an autoclave (121°C, 20 minutes) whereby the ebselen was dissolved completely in the soybean oil.
④ 147 ml of 2.5 % aqueous glycerin solution prepared in item ①was added to the lipid mixture prepared in item ② and emulsified for 10 minutes (preliminary emulsification I) by a TK homo-mixer (Tokushukika Kogyo Co., Ltd.).
⑤To this fluid was gradually added the above solution of ebselen in soybean oil in item ⑤, and the mixture was emulsified for 10 minutes (preliminary emulsification II). The resulting fluid was returned to room temperature and then adjusted to pH 9.03. After the pH adjustment, the fluid was emulsified with a microfluidizer (Microfluidics Co.) at a passing number of 25 (with a coil cooled on ice-cold water), and then its pH was adjusted again (pH 8.97).
⑥After the pH adjustment, the resulting emulsion was pipetted into glass vials (10 ml/vial) and sterilized under the conditions of 121 °C and 20 minutes, whereby the ebselen-containing O/W emulsion preparation of the present invention was obtained.

### EXAMPLE 2

The emulsion preparation of the present invention was prepared in the same manner as described above except that Myglyol was used in place of soybean oil, and the components, amounts and condition described in Table 1 were used. When preliminary emulsification II was finished, the pH was adjusted to 8.95, and after emulsification with the microfluidizer, the pH was adjusted to 8.76.

### REFERENCE EXAMPLE 1

An emulsion preparation was prepared in the same manner as described above except that Myglyol was used in place of soybean oil without using phosphatidylethanolamine and the components, amounts and condition described in Table 1 were used.

### COMPARATIVE EXAMPLES 1 to 5

The emulsion preparations in Comparative Examples 1 to 3 were prepared in the same manner as described above except that the components, amounts and condition described in Table 1 were used. The emulsion preparations in Comparative Examples 4 and 5 were prepared in the same manner as described in Example 14 in JP-A 2-250876 except that the components, amounts and condition described in Table 1 were used.

**Table 1**

| Formulation of Ebselen-Containing Emulsion Preparation/100 ml | | | | | | |
|---|---|---|---|---|---|---|
| | Ebselen (g) | Content of PC¹⁾in egg yolk lecithin (%) | Egg yolk lecithin (g) | PE²⁾ (g) | Fat and lipid (g) | PH adjustment |
| Example 1 | 0.1 | 98 | 1.485 | 0.015 | soybean oil (25 g) | done |
| Example 2 | 0.1 | 98 | 1.425 | 0.075 | Myglyol 812 (25 g) | done |
| Ref. Ex. 1 | 0.1 | 98 | 2.4 | 0 | Myglyol 812 (20 g) | done |
| Com. Ex. 1 | 0.1 | 98 | 1.5 | 0 | soybean oil (25 g) | done |
| Com. Ex. 2 | 0.1 | 98 | 1.425 | 0.075 | soybean oil (25 g) | not done |
| Com. Ex. 3 | 0.1 | 83 | 2.4 | 0 | Myglyol 812 (20 g) | done |
| Com. Ex. 4 | 0.1 | 98 | 2.4 | 0 | Myglyol 812 (20 g) | not done |
| Com. Ex. 5 | 0.1 | 83 | 2.4 | 0 | Myglyol 812 (20 g) | not done |

| | | | | | | |
|---|---|---|---|---|---|---|
| 1) PC: phosphatidylcholine, | | | | | | |
| 2) PE: phosphatidylethanolamine | | | | | | |

### Method of measuring the physical properties of the preparations Appearance

The preparations were observed by viewing.

### Measurement of the content of the active agent

| Measurement by HPLC | |
|---|---|
| [HPLC conditions] | |
| Mobile phase: | phosphate buffer (pH 2.5)/acetonitrile/methanol = 5/5/3 |
| Detector | JASCO 875 UV |
| Detection wavelength | 262 nm |
| Column temperature | a constant temperature in the vicinity of 40 °C |
| Flow rate | 0.8 ml |
| Injection volume | 10 µl |
| Measurement time | 35 minutes |
| Column | YMC-Pack ODS-AM (Eishin Chemical Co., Ltd.) AM-302 150 mmx4.6 mm I.D. |
| Guard column | YMC-Guard pack ODS-AM (Eishin Chemical Co., Ltd.) , BBC-4-ODS-5-AM, 10 mm x 4.0 mm I.D. |

### [Preparation of samples]

### Preparation of an ebselen standard stock solution and an internal standard (IS) solution

50 mg ebselen and 100 mg IS (2,4,5-trichloroaniline) were weighed separately and accurately adjusted respectively to 50 ml with isopropanol.

### Preparation of a sample solution

①About 40 ml isopropanol was placed previously in a measuring flask, and 1 ml emulsion preparation was accurately weighed in a constant delivery pipette and introduced into the measuring flask, and the emulsion preparation still remaining on the inside of the pipette was washed out several times with the isopropanol in the measuring flask.
②1 ml IS was accurately weighed, then introduced into the measuring flask in item ①and adjusted accurately to 50 ml with isopropanol.
③2 ml of the solution prepared in item ②was accurately weighed, then 2 ml mobile phase was accurately added thereto, and the mixture was admixed by shaking.
④The solution prepared in item ③was filtered through a 0.45 µm HV filter to give a sample solution.

### Preparation of a standard solution

③About 40 ml isopropanol was placed previously in a measuring flask, and 1 ml placebo preparation (i.e. an emulsion preparation not containing ebselen) was accurately weighed in a constant delivery pipette and introduced into the measuring flask, and the emulsion preparation still remaining on the inside of the pipette was washed out several times with the isopropanol in the measuring flask.
②1 ml standard stock solution and 1 ml IS were accurately weighed, then introduced into the measuring flask in item ① and adjusted accurately to 50 ml with isopropanol.
③2 ml of the solution prepared in item ② was accurately weighed, then 2 ml mobile phase was accurately added thereto, and the mixture was admixed by shaking.
④The solution prepared in item ③ was filtered through a 0.45 µm HV filter to give a sample solution.

### Particle diameter

The preparation was diluted suitably with 5 % glucose and measured.

### Measuring unit: Particle Sizing Systems, 380ZLS (NICOMP Co., Ltd.) pH

The stock solution was measured as such.

Measuring device: A Φ45 pH meter (Beckman Ltd.)

### Results

The emulsion preparations obtained in the Examples and Comparative Examples were observed and measured for the evaluation items described above, and the results are shown in Table 2.

**Table 2**

| Results of the Ebselen-Containing Preparations (measured upon cooling after heating sterilization) | | | | |
|---|---|---|---|---|
| | Appearance | Content of Ebselen (mg/ml) | Particle diameter (nm) | pH |
| Example 1 | white suspension | 1.08 | 290 | 7.15 |
| Example 2 | white suspension | 0.98 | 229 | 7.07 |
| Ref. Ex. 1 | white suspension | 0.93 | 376 | 6.57 |
| Com. Ex. 1 | separation into two layers | - | 725⁴⁾ | 7.02 |
| Com. Ex. 2 | separation into two layers | - | - | 4.53 |
| Com. Ex. 3 | brown suspension | 0.82³⁾ | 149 | 6.64 |
| Com. Ex. 4 | separation into two layers | - | 2928⁴⁾ | 6.35 |
| Com. Ex. 5 | brown suspension | 0.85³⁾ | 151 | 3.84 |

| | | | | |
|---|---|---|---|---|
| 3): A decomposition peak was recognized. | | | | |
| 4) : Data on the sample before separation into two layers. | | | | |

As can be seen from Table 2 above, the preparations with a pH value of 6 to 8 using egg yolk lecithin having a high content of phosphatidylcholine and containing phosphatidylethanolamine formed a white suspended emulsion with a smaller particle diameter. When Myglyol was used as the fat and oil, the white suspended emulsion could also be obtained even if phosphatidylethanolamine was not used, but its particle diameter was larger than that of the preparations using phosphatidylethanolamine (Reference Example 1).

In contrast, if egg yolk lecithin having a low content of phosphatidylcholine (83 %) was used (Comparative Examples 3 and 5), the appearance of prepared preparations was brown suspension.

On the other hand, if phosphatidylethanolamine was not used (Comparative Example 1), the resulting preparation was separated into the two layers, thus failing to form an emulsion.

And if the pH was not adjusted (Comparative Examples 2 and 4) , the resulting preparations were separated into two layers , thus failing to form an emulsion.

### INDUSTRIAL APPLICABILITY

The preparation of the present invention is excellent in stability as emulsion, and even after heat sterilization, it has excellent stability as emulsion and is excellent as an emulsion preparation.

## Claims

1. An emulsion preparation having a pH value of 6 to 8, comprising ebselen, fat and oil, phosphatidylcholine and phosphatidylethanolamine.

2. The emulsion preparation according to claim 1, wherein the pH value is 6.5 to 7.5.

3. The emulsion preparation according to any one of claims 1 to 2, wherein the ratio of phosphatidylethanolamine and phosphatidylcholine, that is, the ratio of phosphatidylethanolamine : phosphatidylcholine is 0.1 : 99.9 to 10 : 90 (weight ratio).

4. The emulsion preparation according to anyone of claims 1 to 2, wherein the ratio of phosphatidylethanolamine and phosphatidylcholine, that is, the ratio of phosphatidylethanolamine : phosphatidylcholine is 0.1 : 99.9 to 5 : 95 (weight ratio)

5. The emulsion preparation according to any one of claims 1 to 4, wherein the fat and oil are triglycerides.

6. The emulsion preparation according to any one of claims 1 to 4, wherein the fat and oil are soybean oil.

7. The emulsion preparation according to any one of claims 1 to 6, wherein the preparation is a sterile preparation.
